# EUROPÄISCHE PATENTSCHRIFT

(11) **EP 1 846 357 B1**
(45) Veröffentlichungstag und Bekanntmachung des Hinweises auf die Patenterteilung: **03.12.2008**
(21) Anmeldenummer: 06707953.3
(22) Anmeldetag: 01.02.2006
(51) Int. Cl.: C07C 209/64, C07C 211/14

(54) **VERFAHREN ZUR HERSTELLUNG VON BIS-[(3-DIMETHYLAMINO)PROPYL]AMIN (BISDMAPA)**
METHOD FOR PRODUCING BIS-[(3-DIMETHYLAMINO)PROPYL]AMINE (BISDMAPA)
PROCEDE DE PRODUCTION DE BIS-[(3-DIMETHYLAMINO)PROPYL]AMINE(BISDMAPA)

(30) Priorität: 01.02.2005 DE 102005004853
(43) Veröffentlichungstag der Anmeldung: 24.10.2007
(73) Patentinhaber: BASF SE, 67056 Ludwigshafen (DE)
(72) Erfinder: MELDER, Johann-Peter, 67459 Böhl-Iggelheim (DE); KRUG, Thomas, 67550 Worms (DE)
(86) Internationale Anmeldenummer: PCT/EP2006/050591
(87) Internationale Veröffentlichungsnummer: WO 2006/082202

(56) Entgegenhaltungen:
- EP-A- 1 431 273

## Beschreibung

Die vorliegende Erfindung betrifft ein Verfahren zur Herstellung von Bis-[(3-Dimethyl-amino)propyl]amin (BisDMAPA) durch kontinuierliche Umsetzung von 3-(N,N-Dimethylamino)propylamin (DMAPA) in Gegenwart eines Heterogenkatalysators.

BisDMAPA, das die folgende Strukturformel aufweist, findet Verwendung als Zwischenprodukt und zur Synthese von Waschmitteladditiven, PU-Katalysatoren und Korrosionsinhibitoren.

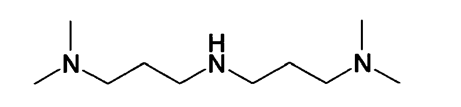

Das als Edukt benötigte 3-(N,N-Dimethylamino)propylamin [(CH₃)₂N-CH₂-CH₂-CH₂-NH₂; DMAPA] kann nach bekannten Verfahren, beispielsweise durch Umsetzung von Acrylnitril mit Dimethylamin (DMA) zu N,N-Dimethylaminopropionitril (DMAPN) und anschließende Hydrierung hergestellt werden.

Symmetrische sekundäre Amine können durch katalytische Aminierung von entsprechenden Alkoholen, Aldehyden oder Ketonen mit entsprechenden primären Aminen unter Freisetzung von einem Moläquivalent Wasser hergestellt werden.

Bekannt sind auch Verfahren zur Herstellung von symmetrischen sekundären Aminen aus primären Aminen durch Dimerisierung des primären Amins in Gegenwart von H₂ unter NH₃-Bildung nach: 2 R-NH₂ + H₂ → R-NH-R + NH₃.

Die Dimerisierung primärer Amine an Übergangsmetall-Katalysatoren zu entsprechenden symmetrischen sekundären Aminen, leidet unter einer Vielzahl von Folgeprodukten und Nebenreaktionen. Sie kann an metallischen Aminierungskatalysatoren (z.B. Ni, Co, Cu) bei erhöhter Temperatur und unter Druck durchgeführt werden.

EP-A1-1 431 273 (BASF AG) betrifft ein Verfahren zur Herstellung eines symmetrischen sekundären Amins durch Umsetzung eines primären Amins in Gegenwart von Wasserstoff und eines Katalysators, wobei man einen Katalysator einsetzt, bei dessen Herstellung eine Fällung von katalytisch aktiven Komponenten auf monoklines, tetragonales oder kubisches Zirkoniumdioxid erfolgte.

EP-A1-1 270 543 (BASF AG) beschreibt ein Verfahren zur Herstellung von bestimmten sekundären Aminen aus primären Aminen in Gegenwart von Wasserstoff und eines Katalysators, der mindestens ein Element oder eine Verbindung eines Elementes aus den Gruppen VIII und IB des Periodensystems enthält. Danach gelingt die Dimerisierung von DMAPA zu BisDMAPA an Ni-haltigen Katalysatoren.

Wegen der Bildung von Ammoniak bei der Konvertierung von DMAPA zu BisDMAPA (2 DMAPA → BisDMAPA + NH₃) gewinnt bei höherem Umsatz zunehmend auch die Rückrektion von BisDMAPA mit Ammoniak zu DMAPA an Bedeutung.

Die WO-Anmeldung PCT/EP/04/014394 vom 17.12.04 (BASF AG) betrifft Verfahren zur Erhöhung der Raum-Zeit-Ausbeute (RZA) in einem Verfahren zur Herstellung eines symmetrischen sekundären Amins durch Umsetzung eines primären Amins in Gegenwart von Wasserstoff und eines Katalysators, indem man unter Beibehaltung der Temperatur den Absolutdruck erniedrigt.

Zur Addition von Alkoholen an Olefine zu entsprechenden Ethern [z.B. MTBE (Methyltertiärbutylether) und TAME (Tertiäramylmethylether)] sind in der Literatur Verfahren bekannt, die in einer Reaktionskolonne durchgeführt werden. Die auch als Reaktivdestillation bezeichneten Verfahren sind z.B. in dem Lehrbuch ,Reactive Distillation', edited by K. Sundmacher und A. Kienle, Verlag Wiley-VCH (2003), ausführlich beschrieben.

Anwendungen der Reaktivdestillation liegen auch auf den Gebieten Veresterungen, Verseifungen und Umesterungen, Herstellung und Verseifung von Acetalen, Herstellung von Alkoholaten, Aldolkondensationen, Alkylierungen, Hydrolyse von Epoxiden, Hydratisierung von Olefinen, Isomerisierungen und Hydrierungen.

Die deutschen Patentanmeldungen Nr. 10336003.4 vom 01.08.03 und Nr. 102004030645.1 vom 24.06.04 (beide BASF AG) betreffen Verfahren zur Herstellung von Ethylenaminen durch kontinuierliche Umsetzung von Ethylendiamin (EDA) in Gegenwart eines Heterogenkatalysators, wobei man die Umsetzung in einer Reaktionskolonne durchführt. Bei den hergestellten Ethylenaminen handelt es sich insbesondere um Diethylentriamin (DETA), Piperazin (PIP) und/oder Triethylentetramin (TETA).

Der vorliegenden Erfindung lag die Aufgabe zugrunde, ein verbessertes wirtschaftliches Verfahren zur selektiven Herstellung von BisDMAPA in hoher Ausbeute und Raum-Zeit-Ausbeute (RZA) aufzufinden.

[Raum-Zeit-Ausbeuten werden angegeben in ,Produktmenge / (Katalysatorvolumen • Zeit)'(kg/(l_{Kat.} • h)) und/oder ,Produktmenge / (Reaktorvolumen • Zeit)' (kg/(l_{Reaktor} • h)].

Demgemäß wurde ein Verfahren zur Herstellung von Bis-[(3-Dimethylamino)propyl]-amin (BisDMAPA) durch kontinuierliche Umsetzung von 3-(N,N-Dimethylamino)-propylamin (DMAPA) in Gegenwart eines Heterogenkatalysators gefunden, welches
dadurch gekennzeichnet ist, dass man die Umsetzung in einer Reaktionskolonne durchführt.

Die Umsetzung im erfindungsgemäßen Verfahren verläuft nach der folgenden Gleichung:

2 DMAPA → BisDMAPA + NH₃

Erfindungsgemäß wurde erkannt, dass Nachteile der Verfahren des Stands der Technik vermieden werden, wenn man die Synthese von BisDMAPA durch kontinuierliche Umsetzung von DMAPA in einer Reaktionskolonne durchführt (Reaktivdestillation). Durch das kontinuierliche Abziehen von BisDMAPA aus der Kolonne unterhalb der Reaktionszone (über Sumpf und/oder über einen optionalen Seitenabzug) können Folgereaktionen weitgehend unterdrückt werden und dadurch wird eine Fahrweise bei hohem Umsatz und sogar Vollumsatz von DMAPA ermöglicht.

Durch das kontinuierliche Entfernen von Ammoniak aus der Kolonne (bevorzugt am Kolonnenkopf, auch als Gemisch mit leichter als BisDMAPA siedenden Komponenten) wird die Rückreaktion von BisDMAPA zu DMAPA weitgehend unterbunden und so die Bildung von BisDMAPA beschleunigt. Die Reaktion kann daher bei anderen Drücken, vorteilhafterweise niedrigeren Drücken, durchgeführt werden, als in dem bei Verwendung eines gewöhnlichen Festbettreaktors (Rohrreaktor mit Katalysatorfestbett) optimalen Druckbereich.

Die Reaktionskolonne weist bevorzugt einen Bereich, in dem die Umsetzung von DMAPA zu BisDMAPA stattfindet (Reaktionszone), einen Verstärkungsteil oberhalb der Reaktionszone und einen Abtriebsteil unterhalb der Reaktionszone auf.

Der Absolutdruck in der Kolonne liegt bevorzugt im Bereich von > 0 bis 20 bar, z.B. im Bereich von 1 bis 20 bar, insbesondere 5 bis 10 bar.

Die Temperatur in dem Bereich der Kolonne, in dem die Umsetzung von DMAPA zu BisDMAPA stattfindet (Reaktionszone), liegt bevorzugt im Bereich von 100 bis 200°C, insbesondere 140 bis 160°C.

Die Zahl der theoretischen Trennstufen in der Kolonne insgesamt liegt bevorzugt im Bereich von 5 bis 100, besonders bevorzugt 10 bis 20.

Die Zahl der theoretischen Trennstufen in der Reaktionszone liegt bevorzugt im Bereich von 1 bis 30, insbesondere 1 bis 20, besonders 1 bis 10, z.B. 5 bis 10.

Die Zahl der theoretischen Trennstufen im Verstärkungsteil oberhalb der Reaktionszone liegt bevorzugt im Bereich von 0 bis 30, besonders 1 bis 30, weiter besonders 1 bis 15, insbesondere 1 bis 5.

Die Zahl der theoretischen Trennstufen im Abtriebsteil unterhalb der Reaktionszone liegt bevorzugt im Bereich von 0 bis 40, besonders 5 bis 30, insbesondere 10 bis 20.

Die Zugabe von DMAPA in die Kolonne kann unterhalb der Reaktionszone in flüssiger Form oder gasförmig erfolgen.
Die Zugabe von DMAPA in die Kolonne kann auch in flüssiger Form oberhalb der Reaktionszone erfolgen.

Im erfindungsgemäßen Verfahren wird bevorzugt reines DMAPA, z.B. in einer Reinheit > 98 Gew.-%, insbesondere > 99 Gew.-%, der Kolonne zugeführt.

Es kann auch das nach teilweiser oder vollständiger Abtrennung von Ammoniak und Wasserstoff erhaltene DMAPA-Rohprodukt aus einer Hydrierung von N,N-Dimethyl-aminopropionitril (DMAPN) eingesetzt werden.

Die Umsetzung wird besonders bevorzugt in Gegenwart von Wasserstoff, insbesondere in Gegenwart von 0,0001 bis 1 Gew.-%, bevorzugt 0,001 bis 0,01 Gew.-%, Wasserstoff, jeweils bezogen auf die Feedmenge an DMAPA, durchgeführt.

Die Zugabe von Wasserstoff in die Kolonne erfolgt bevorzugt unterhalb der Reaktionszone.

Ein Gemisch aus Ammoniak, anderen Komponenten mit einem Siedepunkt tiefer als BisDMAPA (bei gleichen Druck) (Leichtsiedern) und gegebenenfalls Wasserstoff wird bevorzugt über Kopf der Kolonne entnommen.

Das über Kopf der Kolonne entnommene Gemisch kann auch Teilmengen von unumgesetztem DMAPA enthalten.

Das über Kopf entnommene Gemisch kann auch teilweise kondensiert und dabei Ammoniak und gegebenenfalls Wasserstoff überwiegend gasförmig entnommen (abgetrennt) und der verflüssigte Anteil kann als Rücklauf auf die Kolonne gegeben werden.

Das Gewichtsverhältnis der Rücklaufmenge der Kolonne (Kolonnenrücklaufmenge) zur Menge des Zulaufs zur Kolonne liegt dabei bevorzugt im Bereich von 0,1 bis 30, besonders bevorzugt 0,5 bis 10, insbesondere 0,5 bis 2.

Ein Gemisch aus BisDMAPA und anderen Komponenten mit einem Siedepunkt höher als BisDMAPA (bei gleichem Druck) (Schwersiedern), wie z.B. TrisDMAPA [((CH₃)₂NCH₂CH₂CH₂)₃N], wird bevorzugt über Sumpf der Kolonne entnommen.
Das über Sumpf der Kolonne entnommene Gemisch kann auch Teilmengen von unumgesetztem DMAPA oder die Gesamtmenge an unumgesetzten DMAPA enthalten.

In einer besonderen Verfahrensausgestaltung ist die Kolonne unterhalb der Reaktionszone durch einen Seitenabzug unterteilt.
Über den Seitenabzug wird bevorzugt unumgesetztes DMAPA entnommen.

Das über den Seitenabzug entnommene Produkt kann auch BisDMAPA enthalten.

Das über den Seitenabzug anfallende Produkt wird in flüssiger Form oder gasförmig entnommen.

In der Reaktionszone wird als Katalysator bevorzugt ein Katalysator enthaltend Ni, Co, Cu, Ru, Re, Rh, Pd und/oder Pt oder ein formselektiver Zeolithkatalystor oder ein Phosphatkatalysator eingesetzt.

Das oder die Metalle des Übergangsmetallkatalysators, darunter bevorzugt Ru, Re, Rh, Pd und/oder Pt, sind bevorzugt auf einem oxidischen Trägermaterial (z.B. Al₂O₃, TiO₂, ZrO₂, SiO₂) oder auf einem Zeolith oder Aktivkohle als Trägermaterial aufgebracht.

In einer bevorzugten Ausführungsform wird in der Reaktionszone als Katalysator ein Katalysator enthaltend Pd und Zirkoniumdioxid als Trägermaterial eingesetzt.

Der Gesamt-Metallgehalt der Übergangsmetall-Trägerkatalysatoren liegt bevorzugt im Bereich von > 0 bis 80 Gew.-%, besonders 0,1 bis 70 Gew.-%, weiter besonders 5 bis 60 Gew.-%, weiter besonders 10 bis 50 Gew.-%, jeweils bezogen auf das Gewicht des Trägermaterials.

Im Falle der bevorzugten Edelmetall-Trägerkatalysatoren liegt der Gesamt-Edelmetallgehalt insbesondere im Bereich von > 0 bis 20 Gew.-%, besonders 0,1 bis 10 Gew.-%, ganz besonders 0,2 bis 5 Gew.-%, weiter besonders 0,3 bis 2 Gew.-%, jeweils bezogen auf das Gewicht des Trägermaterials.

Die Heterogenkatalysatoren können in Form von festen Katalysatorbetten innerhalb der Kolonne oder in separaten Behältern außerhalb der Kolonne untergebracht werden. Sie können auch als Schüttungen, z.B. als Schüttung in eine Destillationspackung, zum Einsatz kommen, zu Füllkörpern oder Formkörpern geformt werden, beispielsweise zu Raschigringen gepresst, in Filtergewebe eingebracht und zu Rollen (sog. Bales) oder Kolonnenpackungen geformt werden, auf Destillationspackungen aufgebracht werden (Beschichtung) oder als Suspension in der Kolonne, hierbei bevorzugt als Suspension auf Kolonnenböden, eingesetzt werden.

In Verfahren mit heterogen katalysierten Reaktivdestillationen kann vorteilhaft die von der Fa. CDTech entwickelte "bales"-Technologie angewendet werden.

Weitere Technologien sind spezielle Bodenkonstruktionen mit gepackten oder suspendierten Katalysatoren.

Mehrkanalpackungen oder Kreuzkanalpackungen (siehe z.B. WO-A-03/047747) ermöglichen ein einfaches Einfüllen und Austragen von Katalysatoren, die in Teilchenform vorliegen (z.B. Kugeln, Stränglinge, Tabletten), bei geringer mechanischer Belastung des Katalysators.

Ein wichtiger Punkt bei der Reaktivdestillation ist die Bereitstellung der für den Reaktionsablauf benötigten Verweilzeit. Es ist erforderlich, die Verweilzeit der Flüssigkeit in der Kolonne im Vergleich zu einer nichtreaktiven Destillation gezielt zu erhöhen. Man nutzt Sonderkonstruktionen von Kolonneneinbauten, beispielsweise Bodenkolonnen mit Glockenböden mit stark erhöhtem Füllstand, hohe Verweilzeiten in den Ablaufschächten von Bodenkolonnen und/oder auch separat angeordnete außenliegende Verweilzeitbehälter. Anstaupackungen bieten die Möglichkeit, die Verweilzeit der Flüssigkeit um etwa den Faktor 3 gegenüber Füllkörper- und Packungskolonnen zu erhöhen.

Die Auslegung der Reaktionskolonne (z.B. Zahl der Trennstufen in den Kolonnenabschnitten Verstärkungsteil, Abtriebsteil und Reaktionszone, Rücklaufverhältnis, etc.) kann durch den Fachmann nach ihm geläufigen Methoden vorgenommen werden.

Reaktionskolonnen sind in der Literatur z.B. beschrieben in:
'Reactive distillation of nonideal multicomponent mixtures', U. Hoffmann, K. Sundmacher, March 1994, Trondheim/Norway,
'Prozesse der Reaktivdestillation', J. Stichlmair, T. Frey, Chem. Ing. Tech. 70 (1998) 12, Seiten 1507-1516,
'Thermodynamische Grundlagen der Reaktivdestillation', T. Frey, J. Stichlmair, Chem. Ing. Tech. 70 (1998) 11, Seiten 1373-1381,
WO-A-97/16243 vom 09.05.97,
DD-Patent 100701 vom 05.10.73,
US 4,267,396 vom 12.05.81,
'Reaktionen in Destillationskolonnen', G. Kaibel, H.-H. Mayer, B. Seid, Chem. Ing. Tech. 50 (1978) 8, Seiten 586-592, und dort zitierte Literatur,
DE-C2-27 14 590 vom 16.08.84,
EP-B-40724 vom 25.05.83,
EP-B-40723 vom 06.07.83,
DE-C1-37 01 268 vom 14.04.88,
DE-C1-34 13 212 vom 12.09.85,
'Production of potassium tert-butoxide by azeotropic reaction destillation', Wang Huachun, Petrochem. Eng. 26 (1997) 11,
'Design aspects for reactive distillation', J. Fair, Chem. Eng. 10 (1998), Seiten 158-162, EP-B1-461 855 vom 09.08.95,
'Consider reactive distillation', J. DeGarmo, V. Parulekar, V. Pinjala, Chem. Eng. Prog. 3 (1992),
EP-B1-402 019 vom 28.06.95,
'La distillation réaktive', P. Mikitenko, Pétrole et Techniques 329 (1986), Seiten 34-38,
'Geometry and efficiency of reactive distillation bale packing', H. Subawalla, J. González, A. Seibert, J. Fair, Ind. Eng. Chem. Res. 36 (1997), Seiten 3821-3832,
'La distillation réactive', D. Cieutat, Pétrole et Techniques 350 (1989),
'Preparation of tert-amyl alcohol in a reactive distillation column', J. González, H. Subawalla, J. Fair, Ind. Eng. Chem. Res. 36 (1997), Seiten 3845-3853,
'More uses for catalytic distillation', G. Podrebarac, G. Rempel, Chem. Tech. 5 (1997), Seiten 37-45,
'Advances in process technology through catalytic distillation', G. Gildert, K. Rock, T. McGuirk, CDTech, Seiten 103-113,
WO-A1-03/047747 vom 12.06.03 (BASF AG) und
WO-A1-97/35834.

In einer bevorzugten Ausführungsform wird das erfindungsgemäße Verfahren durchgeführt gemäß WO-A1-03/047747 in einer Kolonne zur Durchführung von Reaktivdestillationen in Gegenwart eines heterogenen teilchenförmigen Katalysators, mit einer Packung oder Füllkörpern, die im Kolonneninnenraum Zwischenräume ausbilden, wobei die Kolonne erste und zweite Teilbereiche aufweist, die alternierend angeordnet sind und sich durch die spezifische Oberfläche der Packung oder Füllkörper unterscheiden, dergestalt, dass in den ersten Teilbereichen der Quotient aus dem hydraulischen Durchmesser für den Gasstrom durch die Packung oder Füllkörper und dem äquivalenten Durchmesser der Katalysatorteilchen im Bereich von 2 bis 20, bevorzugt im Bereich von 5 bis 10 liegt, wobei die Katalysatorteilchen lose unter Einwirkung der Schwerkraft in die Zwischenräume eingebracht, verteilt und ausgetragen werden und dass in den zweiten Teilbereichen der Quotient aus dem hydraulischen Durchmesser für den Gasstrom durch die Packung oder die Füllkörper und dem äquivalenten Durchmesser der Katalysatorteilchen kleiner als 1 ist und dass in die zweiten Teilbereiche keine Katalysatorteilchen eingebracht werden. Bevorzugt wird die Kolonne hinsichtlich ihrer Gas- und/oder Flüssigkeitsbelastung so betrieben, dass maximal 50 bis 95 %, bevorzugt 70 bis 80 %, der Flutbelastung erreicht wird. Vergl. loc. cit., Ansprüche 9 und 10.

Die Aufarbeitung der im erfindungsgemäßen Verfahren anfallenden Produktströme, die vor allem das gewünschte BisDMAPA, aber auch ggf. TrisDMAPA und ggf. unumgesetztes DMAPA enthalten, kann nach dem Fachmann bekannten Destillationsverfahren erfolgen. (Vergl. z.B. PEP Report No. 138, "Alkyl Amines", SRI International, 03/1981, Seiten 81-99, 117).

Die zur destillativen Reingewinnung des gewünschten Produkts BisDMAPA benötigten Destillationskolonnen können durch den Fachmann mit ihm geläufigen Methoden ausgelegt werden (z.B. Zahl der Trennstufen, Rücklaufverhältnis, etc.).

Die Fahrweise mit einem Seitenabzug im Abtriebsteil unterhalb der Reaktionszone der Reaktionskolonne bietet besondere Vorteile bei der weiteren Aufarbeitung zur Reingewinnung des BisDMAPAs.

Der Seitenabzugsstrom, bestehend überwiegend aus unumgesetztem DMAPA, enthält nur kleine Mengen an BisDMAPA und Schwersiedern.

Teilmengen oder die gesamte Menge des Seitenstroms können auch in die Reaktionskolonne selbst zurückgeführt werden. Dies ist insbesondere vorteilhaft, wenn der Seitenstrom vorwiegend DMAPA und wenig oder kein BisDMAPA enthält.

Der Sumpfabzugsstrom der Reaktionskolonne enthält bei dieser Fahrweise weniger Leichtsieder (DMAPA), so dass die Kolonne zur Abtrennung der leichtsiedenden Komponenten von BisDMAPA und Schwersiedern entlastet wird.

Wenn die Reaktivdestillation bei kleinen Drücken durchgeführt wird, beispielsweise 1 bis 3 bar, ist es auch möglich, den Sumpfabzugsstrom bei Sumpftemperaturen von etwa 200 bis 240°C frei von DMAPA zu erhalten. Der Sumpfabzugsstrom kann direkt der Reindestillation von BisDMAPA zugeführt werden.

Das erfindungsgemäße Verfahren ermöglicht die Herstellung von BisDMAPA mit einer Selektivität von > 85 %, insbesondere > 90 %, ganz besonders > 95 %, jeweils bezogen auf umgesetztes DMAPA, bei einem DMAPA-Umsatz von > 30 %, insbesondere > 40 %, ganz besonders > 50 %.

### Beispiele

### Beispiel A

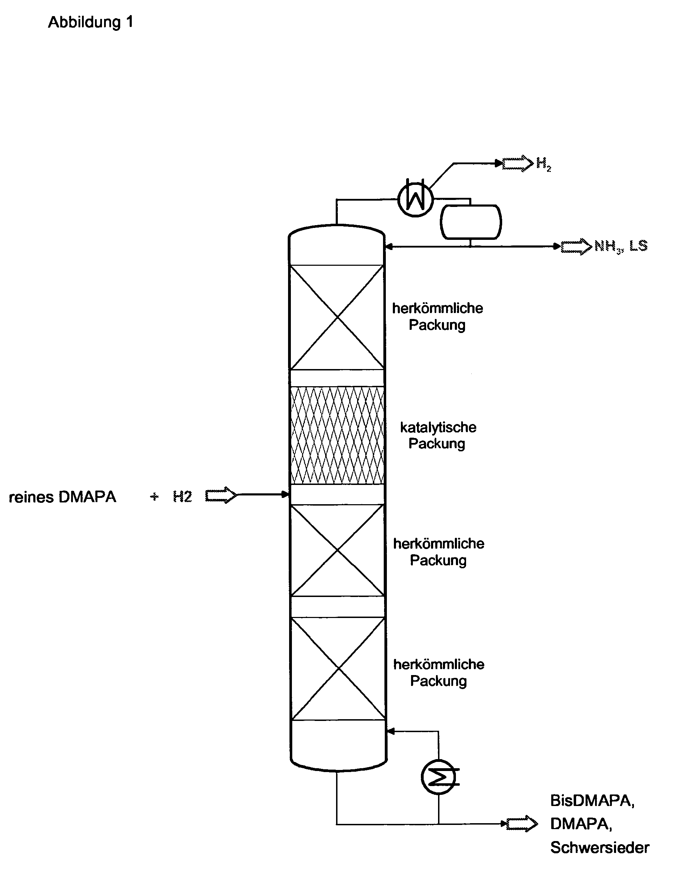

Abbildung 1 in Anlage 1 zeigt eine Ausgestaltung des erfindungsgemäßen Verfahrens bei dem reines DMAPA zusammen mit Wasserstoff der Reaktionskolonne kontinuierlich unterhalb der katalytischen Packung zugeführt wird und ein Gemisch enthaltend BisDMAPA, unumgesetztes DMAPA und Schwersieder (SS, d.h. Komponenten mit einem Siedepunkt höher als BisDMAPA, z.B. TrisDMAPA) über Sumpf erhalten wird. Ammoniak, Wasserstoff und Leichtsieder (LS, d.h. Komponenten mit einem Siedepunkt tiefer als BisDMAPA) werden über Kopf abgetrennt.

### Beispiel B

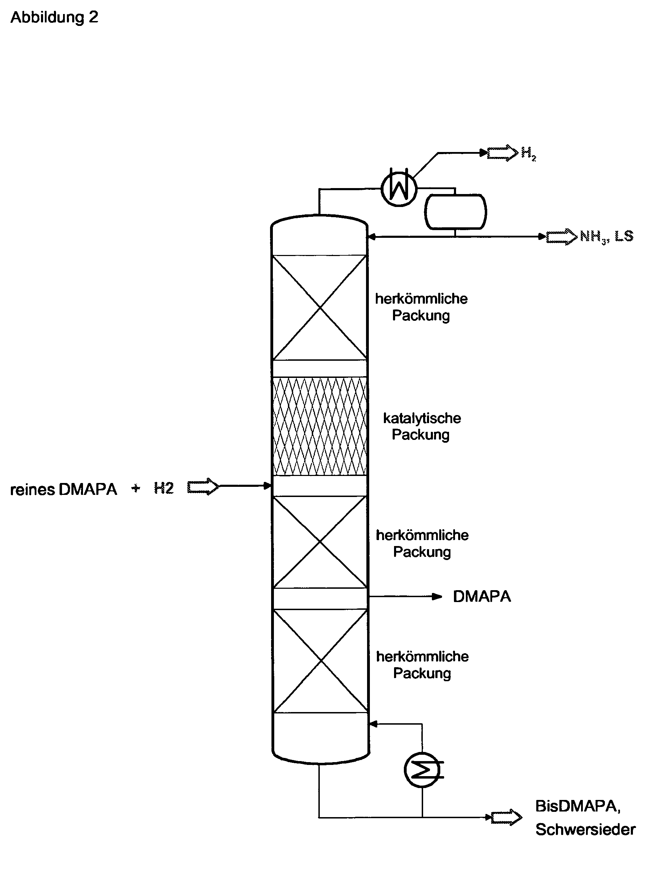

Abbildung 2 in Anlage 2 zeigt eine Ausgestaltung des erfindungsgemäßen Verfahrens bei dem reines DMAPA zusammen mit Wasserstoff der Reaktionskolonne kontinuierlich unterhalb der katalytischen Packung zugeführt wird und ein Gemisch enthaltend BisDMAPA und Schwersieder (SS, d.h. Komponenten mit einem Siedepunkt höher als BisDMAPA, z.B. TrisDMAPA) über Sumpf erhalten wird. Ammoniak, Wasserstoff und Leichtsieder (LS, d.h. Komponenten mit einem Siedepunkt tiefer als BisDMAPA) werden über Kopf abgetrennt.
In einem Seitenabzug im Abtriebsteil unterhalb der Reaktionszone der Reaktionskolonne wird DMAPA abgetrennt.

## Patentansprüche

1. Verfahren zur Herstellung von Bis-[(3-Dimethylamino)propyl]amin (BisDMAPA) durch kontinuierliche Umsetzung von 3-(N,N-Dimethylamino)propylamin (DMAPA) in Gegenwart eines Heterogenkatalysators, **dadurch gekennzeichnet, dass** man die Umsetzung in einer Reaktionskolonne durchführt.

2. Verfahren nach Anspruch 1, **dadurch gekennzeichnet, dass** die Reaktionskolonne mehrere Trennstufen aufweist.

3. Verfahren nach den Ansprüchen 1 oder 2, **dadurch gekennzeichnet, dass** die Reaktionskolonne einen Bereich, in dem die Umsetzung von DMAPA zu BisDMAPA stattfindet (Reaktionszone), einen Verstärkungsteil oberhalb der Reaktionszone und einen Abtriebsteil unterhalb der Reaktionszone aufweist.

4. Verfahren nach einem der vorhergehenden Ansprüche, **dadurch gekennzeichnet, dass** der Absolutdruck in der Kolonne im Bereich von > 0 bis 20 bar liegt.

5. Verfahren nach einem der vorhergehenden Ansprüche, **dadurch gekennzeichnet, dass** die Temperatur in der Reaktionszone im Bereich von 100 bis 200°C liegt.

6. Verfahren nach einem der vorhergehenden Ansprüche, **dadurch gekennzeichnet, dass** die Zahl der theoretischen Trennstufen in der Kolonne insgesamt im Bereich von 5 bis 100 liegt.

7. Verfahren nach einem der vorhergehenden Ansprüche, **dadurch gekennzeichnet, dass** die Zahl der theoretischen Trennstufen in der Reaktionszone im Bereich von 1 bis 30 liegt.

8. Verfahren nach einem der vorhergehenden Ansprüche, **dadurch gekennzeichnet, dass** die Zahl der theoretischen Trennstufen im Verstärkungsteil oberhalb der Reaktionszone im Bereich von 0 bis 30 liegt.

9. Verfahren nach einem der vorhergehenden Ansprüche, **dadurch gekennzeichnet, dass** die Zahl der theoretischen Trennstufen im Abtriebsteil unterhalb der Reaktionszone im Bereich von 0 bis 40 liegt.

10. Verfahren nach einem der vorhergehenden Ansprüche, **dadurch gekennzeichnet, dass** in der Reaktionszone als Katalysator ein Katalysator enthaltend Ni, Co, Cu, Ru, Re, Rh, Pd und/oder Pt oder ein formselektiver Zeolithkatalystor oder ein Phosphatkatalysator eingesetzt wird.

11. Verfahren nach einem der vorhergehenden Ansprüche, **dadurch gekennzeichnet, dass** in der Reaktionszone als Katalysator ein Katalysator enthaltend Pd und Zirkoniumdioxid als Trägermaterial eingesetzt wird.

12. Verfahren nach einem der vorhergehenden Ansprüche, **dadurch gekennzeichnet, dass** der Katalysator als Schüttung in die Reaktionskolonne eingebracht ist.

13. Verfahren nach einem der vorhergehenden Ansprüche, **dadurch gekennzeichnet, dass** der Katalysator als Schüttung in eine Destillationspackung eingebracht ist.

14. Verfahren nach einem der Ansprüche 1 bis 11, **dadurch gekennzeichnet, dass** der Katalysator als Beschichtung auf einer Destillationspackung vorliegt.

15. Verfahren nach einem der Ansprüche 1 bis 11, **dadurch gekennzeichnet, dass** der Katalysator in einem außerhalb der Kolonne befindlichen Verweilzeitbehälter vorliegt.

16. Verfahren nach einem der vorhergehenden Ansprüche, **dadurch gekennzeichnet, dass** die Zugabe von DMAPA in die Kolonne in flüssiger Form unterhalb der Reaktionszone erfolgt.

17. Verfahren nach einem der Ansprüche 1 bis 15, **dadurch gekennzeichnet, dass** die Zugabe von DMAPA in die Kolonne gasförmig unterhalb der Reaktionszone erfolgt.

18. Verfahren nach einem der Ansprüche 1 bis 15, **dadurch gekennzeichnet, dass** die Zugabe von DMAPA in die Kolonne in flüssiger Form oberhalb der Reaktionszone erfolgt.

19. Verfahren nach einem der vorhergehenden Ansprüche, **dadurch gekennzeichnet, dass** das DMAPA der Kolonne in einer Reinheit > 98 Gew.-% zugeführt wird.

20. Verfahren nach einem der vorhergehenden Ansprüche, **dadurch gekennzeichnet, dass** die Umsetzung in Gegenwart von Wasserstoff durchgeführt wird.

21. Verfahren nach dem vorhergehenden Anspruch, **dadurch gekennzeichnet, dass** die Umsetzung in Gegenwart von 0,0001 bis 1 Gew.-% Wasserstoff bezogen auf die Feedmenge an DMAPA durchgeführt wird.

22. Verfahren nach einem der beiden vorhergehenden Ansprüche, **dadurch gekennzeichnet, dass** die Zugabe von Wasserstoff in die Kolonne unterhalb der Reaktionszone erfolgt.

23. Verfahren nach einem der vorhergehenden Ansprüche, **dadurch gekennzeichnet, dass** über Kopf der Kolonne ein Gemisch aus Ammoniak, anderen Komponenten mit einem Siedepunkt tiefer als BisDMAPA (Leichtsiedern) und gegebenenfalls Wasserstoff entnommen wird.

24. Verfahren nach dem vorhergehenden Anspruch, **dadurch gekennzeichnet, dass** das über Kopf der Kolonne entnommene Gemisch auch Teilmengen von unumgesetztem DMAPA enthält.

25. Verfahren nach einem der beiden vorhergehenden Ansprüche, **dadurch gekennzeichnet, dass** das über Kopf entnommene Gemisch teilweise kondensiert wird und dabei Ammoniak und gegebenenfalls Wasserstoff überwiegend gasförmig entnommen werden und der verflüssigte Anteil als Rücklauf auf die Kolonne gegeben wird.

26. Verfahren nach einem der vorhergehenden Ansprüche**, dadurch gekennzeichnet, dass** das Gewichtsverhältnis der Rücklaufmenge der Kolonne zur Menge des Zulaufs zur Kolonne im Bereich von 0,1 bis 30 liegt.

27. Verfahren nach einem der vorhergehenden Ansprüche, **dadurch gekennzeichnet, dass** über Sumpf der Kolonne ein Gemisch aus BisDMAPA und anderen Komponenten mit einem Siedepunkt höher als BisDMAPA (Schwersiedem) entnommen wird.

28. Verfahren nach dem vorhergehenden Anspruch, **dadurch gekennzeichnet, dass** das über Sumpf der Kolonne entnommene Gemisch auch Teilmengen von unumgesetztem DMAPA oder die Gesamtmenge an unumgesetzten DMAPA enthält.

29. Verfahren nach einem der vorhergehenden Ansprüche, **dadurch gekennzeichnet, dass** die Kolonne unterhalb der Reaktionszone durch einen Seitenabzug unterteilt ist.

30. Verfahren nach dem vorhergehenden Anspruch, **dadurch gekennzeichnet, dass** über den Seitenabzug unumgesetztes DMAPA entnommen wird.

31. Verfahren nach einem der beiden vorhergehenden Ansprüche, **dadurch gekennzeichnet, dass** über den Seitenabzug entnommenes Produkt BisDMAPA enthält.

32. Verfahren nach einem der drei vorhergehenden Ansprüche, **dadurch gekennzeichnet, dass** über den Seitenabzug anfallendes Produkt in flüssiger Form entnommen wird.

33. Verfahren nach einem der Ansprüche 29 bis 31, **dadurch gekennzeichnet, dass** über den Seitenabzug anfallendes Produkt gasförmig entnommen wird.

34. Verfahren nach einem der vorhergehenden Ansprüche zur Herstellung von BisDMAPA mit einer Selektivität von > 90 %, bezogen auf DMAPA, bei einem DMAPA-Umsatz von > 50 %.

## Claims

1. A process for preparing bis[(3-dimethylamino)propyl]amine (BisDMAPA) by continuous reaction of 3-(N,N-dimethylamino)propylamine (DMAPA) in the presence of a heterogeneous catalyst, wherein the reaction is carried out in a reaction column.

2. The process according to claim 1, wherein the reaction column has a plurality of theoretical plates.

3. The process according to claim 1 or 2, wherein the reaction column has a region in which the conversion of DMAPA into bisDMAPA takes place (reaction zone), an enrichment section above the reaction zone and a stripping section below the reaction zone.

4. The process according to any of the preceding claims, wherein the absolute pressure in the column is in the range from > 0 to 20 bar.

5. The process according to any of the preceding claims, wherein the temperature in the reaction zone is in the range from 100 to 200°C.

6. The process according to any of the preceding claims, wherein the total number of theoretical plates in the column is in the range from 5 to 100.

7. The process according to any of the preceding claims, wherein the number of theoretical plates in the reaction zone is in the range from 1 to 30.

8. The process according to any of the preceding claims, wherein the number of theoretical plates in the enrichment section above the reaction zone is in the range from 0 to 30.

9. The process according to any of the preceding claims, wherein the number of theoretical plates in the stripping section below the reaction zone is in the range from 0 to 40.

10. The process according to any of the preceding claims, wherein the catalyst used in the reaction zone is a catalyst comprising Ni, Co, Cu, Ru, Re, Rh, Pd and/or Pt or a shape-selective zeolite catalyst or at phosphate catalyst.

11. The process according to any of the preceding claims, wherein the catalyst used in the reaction zone is a catalyst comprising Pd and zirconium dioxide as support material.

12. The process according to any of the preceding claims, wherein the catalyst is installed as a bed in the reaction column.

13. The process according to any of the preceding claims, wherein the catalyst is installed as a bed in a distillation packing.

14. The process according to any of claims 1 to 11, wherein the catalyst is present as a coating on a distillation packing.

15. The process according to any of claims 1 to 11, wherein the catalyst is present in a residence vessel located outside the column.

16. The process according to any of the preceding claims, wherein the DMAPA is introduced into the column in liquid form below the reaction zone.

17. The process according to any of claims 1 to 15, wherein the DMAPA is introduced into the column in gaseous form below the reaction zone.

18. The process according to any of claims 1 to 15, wherein the DMAPA is introduced into the column in liquid form above the reaction zone.

19. The process according to any of the preceding claims, wherein the DMAPA is fed into the column in a purity of > 98% by weight.

20. The process according to any of the preceding claims, wherein the reaction is carried out in the presence of hydrogen.

21. The process according to any of the preceding claims, wherein the reaction is carried out in the presence of from 0.0001 to 1% by weight of hydrogen based on the amount of DMAPA fed in.

22. The process according to either of the two preceding claims, wherein the hydrogen is introduced into the column below the reaction zone.

23. The process according to any of the preceding claims, wherein a mixture of ammonia, other components having a boiling point lower than that of bisDMAPA (low boilers) and possibly hydrogen is taken off at the top of the column.

24. The process according to any of the preceding claims, wherein the mixture taken off at the top of the column further comprises partial amounts of unreacted DMAPA.

25. The process according to either of the two preceding claims, wherein the mixture taken off at the top is partially condensed and ammonia and any hydrogen are predominantly taken off in gaseous form and the liquefied fraction is returned to the column as runback.

26. The process according to any of the preceding claims, wherein the weight ratio of the amount of runback introduced into the column to the amount of feed introduced into the column is in the range from 0.1 to 30.

27. The process according to any of the preceding claims, wherein a mixture of bisDMAPA and other components having a boiling point higher than that of bisDMAPA (high boilers) is taken off from the bottom of the column.

28. The process according to the preceding claim, wherein the mixture taken off at the bottom of the column further comprises partial amounts of unreacted DMAPA or the total amount of unreacted DMAPA.

29. The process according to any of the preceding claims, wherein the column is divided by means of a side offtake below the reaction zone.

30. The process according to the preceding claim, wherein unreacted DMAPA is taken off via the side offtake.

31. The process according to either of the two preceding claims, wherein product taken off via the side offtake comprises bisDMAPA.

32. The process according to any of the three preceding claims, wherein product obtained via the side offtake is taken off in liquid form.

33. The process according to any of claims 29 to 31, wherein product obtained via the side offtake is taken off in gaseous form.

34. The process according to any of the preceding claims for preparing bisDMAPA with a selectivity of > 90%, based on DMAPA, at a DMAPA conversion of > 50%.

## Revendications

1. Procédé de fabrication de bis-[(3-diméthylamino)-propyl]amine (bisDMAPA) par transformation continue de 3-(N,N-diméthylamino)propylamine (DMAPA) en présence d'un catalyseur hétérogène, **caractérisé en ce que** la transformation est réalisée dans une colonne de réaction.

2. Procédé selon la revendication 1, **caractérisé en ce que** la colonne de réaction comprend plusieurs plateaux de séparation.

3. Procédé selon la revendication 1 ou 2, **caractérisé en ce que** la colonne de réaction comprend une zone dans laquelle la transformation de DMAPA en bisDMAPA a lieu (zone de réaction), une partie d'enrichissement au-dessus de la zone de réaction et une partie d'appauvrissement au-dessous de la zone de réaction.

4. Procédé selon l'une quelconque des revendications précédentes, **caractérisé en ce que** la pression absolue dans la colonne est de > 0 à 20 bars.

5. Procédé selon l'une quelconque des revendications précédentes, **caractérisé en ce que** la température dans la zone de réaction est de 100 à 200 °C.

6. Procédé selon l'une quelconque des revendications précédentes, **caractérisé en ce que** le nombre de plateaux de séparation théoriques de la colonne est au total de 5 à 100.

7. Procédé selon l'une quelconque des revendications précédentes, **caractérisé en ce que** le nombre de plateaux de séparation théoriques dans la zone de réaction est de 1 à 30.

8. Procédé selon l'une quelconque des revendications précédentes, **caractérisé en ce que** le nombre de plateaux de séparation théoriques dans la partie d'enrichissement au-dessus de la zone de réaction est de 0 à 30.

9. Procédé selon l'une quelconque des revendications précédentes, **caractérisé en ce que** le nombre de plateaux de séparation théoriques dans la partie d'appauvrissement au-dessous de la zone de réaction est de 0 à 40.

10. Procédé selon l'une quelconque des revendications précédentes, **caractérisé en ce qu'**un catalyseur contenant Ni, Co, Cu, Ru, Re, Rh, Pd et/ou Pt ou un catalyseur à base de zéolithe sélectif de la forme ou un catalyseur à base de phosphate est utilisé en tant que catalyseur dans la zone de réaction.

11. Procédé selon l'une quelconque des revendications précédentes, **caractérisé en ce qu'**un catalyseur contenant du Pd et du dioxyde de zirconium en tant que matériau support est utilisé en tant que catalyseur dans la zone de réaction.

12. Procédé selon l'une quelconque des revendications précédentes, **caractérisé en ce que** le catalyseur est introduit dans la colonne de réaction sous forme d'un granulat.

13. Procédé selon l'une quelconque des revendications précédentes, **caractérisé en ce que** le catalyseur est introduit dans un garnissage de distillation sous forme d'un granulat.

14. Procédé selon l'une quelconque des revendications 1 à 11, **caractérisé en ce que** le catalyseur est présent sous forme d'un revêtement sur un garnissage de distillation.

15. Procédé selon l'une quelconque des revendications 1 à 11, **caractérisé en ce que** le catalyseur est présent dans un conteneur de rétention se trouvant à l'extérieur de la colonne.

16. Procédé selon l'une quelconque des revendications précédentes, **caractérisé en ce que** l'introduction de DMAPA dans la colonne a lieu sous forme liquide au-dessous de la zone de réaction.

17. Procédé selon l'une quelconque des revendications 1 à 15, **caractérisé en ce que** l'introduction de DMAPA dans la colonne a lieu sous forme gazeuse au-dessous de la zone de réaction.

18. Procédé selon l'une quelconque des revendications 1 à 15, **caractérisé en ce que** l'introduction de DMAPA dans la colonne a lieu sous forme liquide au-dessus de la zone de réaction.

19. Procédé selon l'une quelconque des revendications précédentes, **caractérisé en ce que** la DMAPA de la colonne est introduite avec une pureté > 98 % en poids.

20. Procédé selon l'une quelconque des revendications précédentes, **caractérisé en ce que** la transformation est réalisée en présence d'hydrogène.

21. Procédé selon la revendication précédente, **caractérisé en ce que** la transformation est réalisée en présence de 0,0001 à 1 % en poids d'hydrogène par rapport à la quantité de DMAPA introduite.

22. Procédé selon l'une quelconque des deux revendications précédentes, **caractérisé en ce que** l'introduction d'hydrogène dans la colonne a lieu au-dessous de la zone de réaction.

23. Procédé selon l'une quelconque des revendications précédentes, **caractérisé en ce qu'**un mélange d'ammoniac, d'autres composants ayant un point d'ébullition inférieur à la bisDMAPA (composés à point d'ébullition bas) et éventuellement d'hydrogène est prélevé à la tête de la colonne.

24. Procédé selon la revendication précédente, **caractérisé en ce que** le mélange prélevé à la tête de la colonne contient également des parties de DMAPA non réagie.

25. Procédé selon l'une quelconque des deux revendications précédentes, **caractérisé en ce que** le mélange prélevé à la tête est partiellement condensé et ainsi de l'ammoniac et éventuellement de l'hydrogène sont prélevés sous forme principalement gazeuse et la partie liquéfiée est introduite dans la colonne en tant que reflux.

26. Procédé selon l'une quelconque des revendications précédentes, **caractérisé en ce que** la proportion en poids de la quantité de reflux de la colonne par rapport à la quantité de l'alimentation de la colonne est de 0,1 à 30.

27. Procédé selon l'une quelconque des revendications précédentes, **caractérisé en ce qu'**un mélange de bisDMAPA et d'autres composants ayant un point d'ébullition supérieur à la bisDMAPA (composés à point d'ébullition élevé) est prélevé en bas de la colonne.

28. Procédé selon la revendication précédente, **caractérisé en ce que** le mélange prélevé en bas de la colonne contient également des parties de DMAPA non réagie ou la totalité de DMAPA non réagie.

29. Procédé selon l'une quelconque des revendications précédentes, **caractérisé en ce que** la colonne est subdivisée par une évacuation latérale sous la zone de réaction.

30. Procédé selon la revendication précédente, **caractérisé en ce que** de la DMAPA non réagie est prélevée au niveau de l'évacuation latérale.

31. Procédé selon l'une quelconque des deux revendications précédentes, **caractérisé en ce que** le produit prélevé au niveau de l'évacuation latérale contient de la bisDMAPA.

32. Procédé selon l'une quelconque des trois revendications précédentes, **caractérisé en ce que** le produit apparaissant au niveau de l'évacuation latérale est prélevé sous forme liquide.

33. Procédé selon l'une quelconque des revendications 29 à 31, **caractérisé en ce que** le produit apparaissant au niveau de l'évacuation latérale est prélevé sous forme gazeuse.

34. Procédé selon l'une quelconque des revendications précédentes pour la fabrication de bisDMAPA avec une sélectivité > 90 %, par rapport à la DMAPA, avec une conversion de DMAPA > 50 %.
